(19) **Europäisches Patentamt / European Patent Office / Office européen des brevets**

(11) **EP 1 660 156 B1**

(12) **EUROPEAN PATENT SPECIFICATION**

(45) Date of publication and mention
of the grant of the patent:
**10.12.2008 Bulletin 2008/50**

(51) Int Cl.:
*A61M 5/142* (2006.01)    *A61M 5/14* (2006.01)
*F16B 2/20* (2006.01)

(21) Application number: **04781959.4**

(22) Date of filing: **24.08.2004**

(86) International application number:
**PCT/US2004/027371**

(87) International publication number:
**WO 2005/021069 (10.03.2005 Gazette 2005/10)**

(54) **SYRINGE PUMP COMPRISING AN ANTI-ROTATION CLAMP**

SPRITZENPUMPE MIT ANTI-ROTATIONS-KLEMME

POMPE DE SERINGUE COMPRENANT UN CLAMP ANTI-ROTATION

(84) Designated Contracting States:
**AT BE BG CH CY CZ DE DK EE ES FI FR GB GR HU IE IT LI LU MC NL PL PT RO SE SI SK TR**

(30) Priority: **25.08.2003 US 497342 P**

(43) Date of publication of application:
**31.05.2006 Bulletin 2006/22**

(73) Proprietor: **GEN-PROBE INCORPORATED**
**San Diego, CA 92121-1589 (US)**

(72) Inventors:
• **KENNEDY, Mark, R.**
**San Diego, CA 92128 (US)**

• **KNIGHT, Byron**
**San Diego, CA 92107 (US)**

(74) Representative: **Viering, Jentschura & Partner**
**Postfach 22 14 43**
**80504 München (DE)**

(56) References cited:
**CH-A- 269 995**          **FR-A- 2 278 006**
**GB-A- 1 297 794**        **US-A- 5 014 939**
**US-A- 5 219 099**        **US-A- 5 881 438**
**US-A- 2002 173 809**     **US-A1- 2004 049 892**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The invention relates to a syringe pump assembly according to the precharacterizing part of claim 1

The present invention implies a clamp which relies on the elastic force inherent In its shape and the material of which it is made to thereby exert a uniform and controlled force on a structure held thereby.

## Description of the Related Art

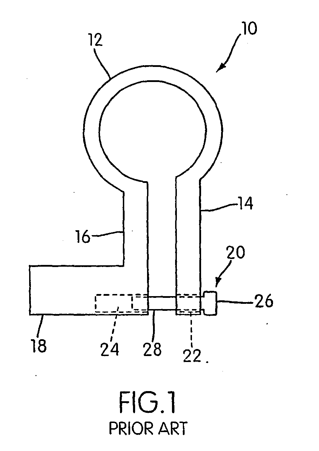

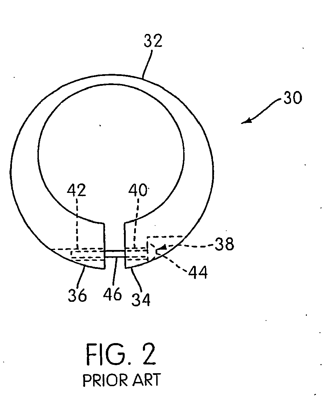

Known clamp devices suitable for holding tubes or hoses are described below and are depleted in Figs. 1 and 2. Fig. 1 shows a prior art clamp 10 comprising a curved resilient portion 12. Extending from the curved resilient portion 12 is a first post 14 with an unthreaded hole 22 located at the free end of first post 14. Also extending from an opposite end of the curved resilient portion 12 is a second post 16, and extending off of the end of the second post 16 is a projection 18. The projection 18 has a threaded hole 24 coaxially aligned with and directly opposed to the unthreaded hole 22 found at the free end of first post 14. The clamp 10 also has a screw 20 having a head 26 and threaded shaft 28. The shaft 28 of screw 20 is inserted through the unthreaded hole 22 and then screwed into the threaded hole 24. As the screw 20 is tightened into hole 24, head 26 engages post 14 forcing the two posts 14 and 16 together, thereby causing the curved resilient portion 12 to deflect in such a manner that its radius will decrease. Deflection of the curved resilient portion 12 will cause it to clamp down on any tubular structure that it may hold. Likewise, when the screw 20 is loosened the curved resilient portion 12, assuming that the aforementioned deflection is elastic, will expand as the two posts 14 and 16 move away from each other.

Fig. 2 shows a circular clamp 30 comprising a circular arc portion 32 having a first end 34 and a second end 36. An unthreaded hole 40 is provided in the first end 34 of the circular clamp 30, and a threaded hole 42, which is coaxially aligned with hole 40, is provided in the second end 36 of the circular clamp 30. Furthermore, the circular clamp 30 also has a screw 38 having a threaded shaft 46 and a head 44. The shaft 46 of screw 38 is first inserted through the unthreaded hole 40 and then screwed into the threaded hole 42. As the screw 38 is tightened, the head 44 engages the first end 34, thereby forcing the first and second ends 34 and 36 of the circular clamp 30 together. This causes the circular arc portion 32 of the circular clamp 30 to contract radially and clamp down on any tubular structure that it may hold. Likewise, when the screw 38 is loosened, the circular arc portion 32 will expand as the two ends 34 and 36 move away from each other.

While the two clamps illustrated in Figs. 1 and 2 may be able to hold certain tubular structures, these clamps lack a mechanism for accurately limiting the amount of force they exert on such structures. Such a force-limiting mechanism is an important feature that acts to prevent excessive generation of clamping forces, which can cause breakage, cracking, and/or buckling of brittle or pliable tubular structures held therein. The illustrated prior art clamps are tightened down as the screw is tightened without any means for limiting the amount of contraction and force exerted by the clamp as a result of the screw being tightened. While these prior art clamps may be suited for strong tubular structures made from materials which can sustain relatively large clamping forces without buckling or breaking, such as steel or thick plastic, they are not suited for tubular structures, such as those made of brittle materials such as glass, or pliable materials such as aluminum, which are delicate and can be easily cracked, broken or buckled if subjected to large clamping forces.

For example, one application of a tubular clamp that requires a controlled clamping force on a delicate Item is with syringe pumps commonly used in laboratory and medical instruments. Such pumps include syringes having tubular portions - , known as barrels - that are constructed of ground glass, which is a very delicate material vulnerable to cracking when subjected to point contact forces. Clamps are frequently employed in these instruments as anti-rotation devices to prevent the syringes from unthreading and losing vacuum during cycling of the pumps and vibration of the Instruments. However, without a force limiting mechanism, the clamps can cause the delicate glass barrels of the syringes to crack, resulting In leaks and wasted material.

For the foregoing reasons, there is a need for a clamp apparatus which evenly distributes the force It exerts on generally cylindrical structures and also has a bullt-in load limitation mechanism, which enables it to hold delicate, generally cylindrical structures in a snug fashion without causing them to break, crack, or otherwise be deformed during installation or use.

Various clamps for different purposes are known for example from documents CH 269955, FR-A-2278006, and US 2002/173809. These clamps widen when pressing two finger grip parts together. Further, from US-A-5219099 a syringe pump is known.

It is an object of the invention to create syringe pump assembly as defined in the precharacterizing part of claim 1 wherein a mechanized syringe is held in such a manner that a rotation of the syringe during operation of the syringe pump is effectively prevented, but without the risk of damaging the syringe.

This is achieved by the features in the characterizing part of claim 1. Preferred embodiments are claimed in claims 2 to 17.

The structure of the present invention provides a number of advantages. For example, in syringe pump applications the clamp is constructed and arranged to provide sufficient holding torque to resist rotation of the syringe while preventing over-tightening of the clamp on the syringe during clamp installation, thereby preventing cracking of the glass barrels. Additionally, the present invention provides a novel design which allows obvious orientation for installation, making it efficient and easy to use.

The invention will be described in detail with reference to the following drawings, In which like features are represented by common reference numbers and in which:

Fig. 1 illustrates a first prior art clamp;

Fig. 2 Illustrates a second prior art clamp;

Fig. 3A is a plan view of a clamp for use in a preferred embodiment of the present invention;

Fig. 3B is a partial view of the portion of Fig. 3A inside oval B;

Fig. 3C is a cross-sectional view of the clamp taken in the direction B-B in Fig. 3A;

Fig. 3D is a perspective view of the clamp of Fig. 3A;

Fig. 4 Is a plan view of a clamp according to a preferred embodiment for use in the present invention Illustrating representative dimensions;

Fig. 5 is a perspective view of a syringe pump for use in the present invention; and

Fig. 6 is a partial perspective view of a clamp installed on a syringe of a syringe pump according to the present invention.

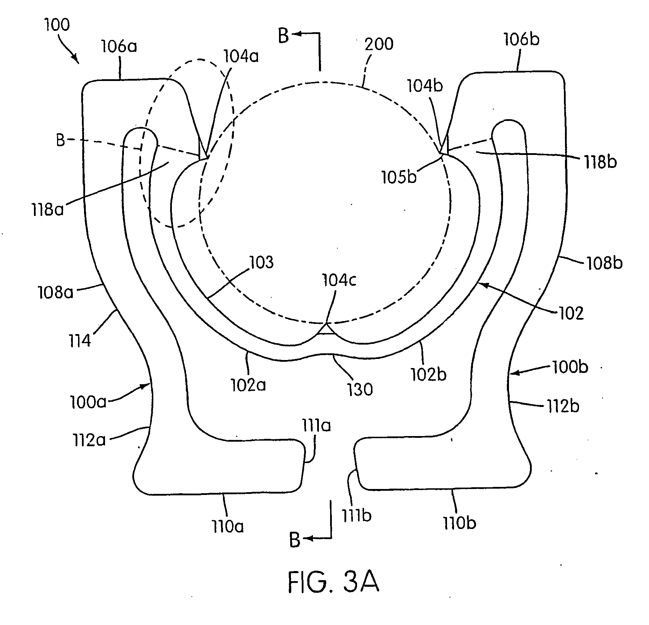

Figs. 3A-3D illustrate a clamp according to a preferred embodiment to be used in the present invention generally indicated by reference number 100, Referring to Fig. 3A, the clamp 100 has a clamp body that is generally symmetrical about a planar section B-B and has two opposing sections 100a and 100b, which mirror one another with reference to plane B-B, Each section 100a, 100b includes an arcuate section 102a, 102b, which together form a hoop section 102 that defines a generally circular cavity for receiving an object (e.g., cylindrical object 200 - which may be a solid cylindrical element or a hollow (i.e., tubular) cylindrical element - shown in Fig. 3A in phantom) to be clamped. Hoop section 102 may also Include anti-slipping elements formed along an interior surface 103 thereof which are constructed and arranged to prevent slippage of the clamp 100 with respect to an object being clamped. For example, the anti-slipping elements may comprise a number of circumferentially-spaced radial projections 104a, 104b, 104c disposed along the interior surface 103 of hoop section 102 for contacting the object 200 being clamped. The projections, which are preferably pointed, are advantageous for holding objects with a knurled surface as the pointed projections will physically engage the peaks and recesses of the knurled surface thereby providing mechanical, non-slip engagement between the clamp and the object being clamped. Alternative anti-slipping elements include a gripping surface material, such as a rubber film provided on at least a portion of the interior surface 103 of the hoop section 102, or knurling or other texture or surface irregularities formed on the interior surface 103 of the hoop section 102.

In the illustrated embodiment, clamp 100 is shown having three radial projections 104a, 104b, 104c, although the clamp 100 may have a different number of projections. Three projections are preferred, however, because they provide 3-point stable contact with an object being clamped, especially if the object is not perfectly round.

Projections 104a, 104b, 104c can be generally triangular in shape. Two projections 104a, 104b are disposed at first and second ends 118a, 118b, respectively, of the hoop section 102, and a center projection 104c is disposed where axis B-B intersects the hoop section 102, at a circumferential mid point between the projections 104a, 104b. Projections 104a, 104b, 104c preferably have chamfered corners 105a, 105b, 105c, respectively, to reduce the sharpness of the corners.

The clamp 100 further includes exterior arms, or actuating elements, 108a, 108b coupled with the arcuate sections 102a, 102b, respectively, via elbows, or coupling means, 106a, 106b, respectively. More specifically, the exterior arm 108a, 108b are coupled to the ends 118a, 118b, respectively, of the hoop section 102 corresponding to projections 104a, 104b and extend along arcuate sections 102a and 102b, respectively. Exterior arms 108a, 108b define finger-operable actuating elements, for opening the clamp by squeezing the exterior arms toward one another between a user's thumb and forefinger. For that purpose, exterior arms 108a, 108b preferably include inwardly curved portions, known as finger grips, as shown at 112a and 112b, to allow easier gripping and squeezing. Each exterior arm 108a, 108b may have an expansion-limiting extension 110a, 110b disposed at the respective ends thereof opposite elbows 106a, 106b. The purpose and functionality of the extensions 110a, 110b will be described below.

Applying an inward force to the finger grips 112a, 112b, such as by squeezing or otherwise moving the free ends of the exterior arms 108a, 108b toward one another, causes radial expansion of the hoop section 102, thereby increasing the size of the gap between projections 104a, 104b. With the size of the gap sufficiently increased, an object 200 to be clamped can be passed through the gap and into the cavity defined by the interior surface of the hoop section 102. When the exterior arms 108a, 108b are permitted to return to their original positions, the elasticity of the hoop section 102 causes the hoop section 102 to grip the object placed in the hoop section 102. Of course, for the elastic restoring forces of the hoop section 102 to generate a clamping force on the object 200, the outside diameter of the object 200 must larger than the inside diameter of the hoop section 102, as defined at the peaks of the projections 104a, 104b, 104c. Because the maximum clamping force that can be generated by the hoop section 102 is defined and limited by the elasticity of the hoop section 102, there is no opportunity for a user to over-tighten the clamp by trying to generate additional clamping force.

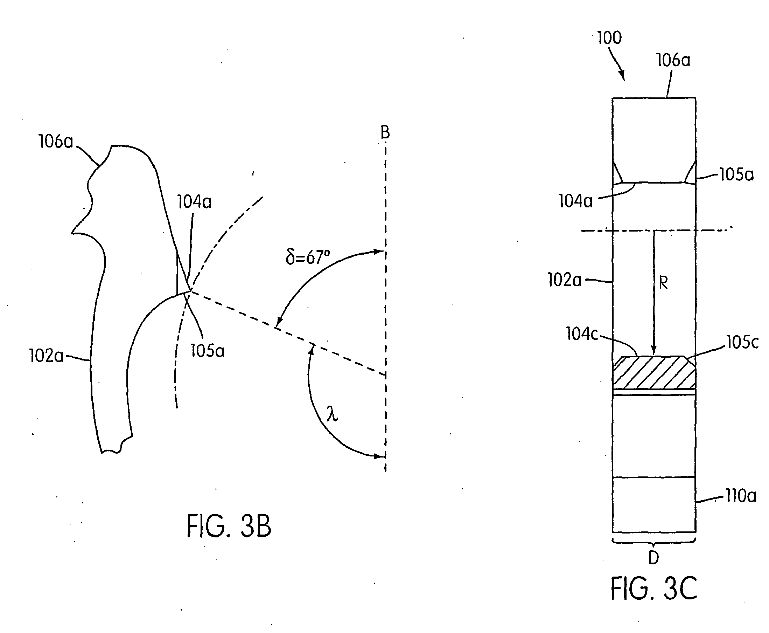

As shown, projections 104a, 104b, 104c are disposed along the interior 103 of the hoop section 102 at circumferentially-spaced positions so as to provide a secure, three-point clamping force on object 200 to be held by clamp 100. As illustrated in Fig. 3B, the end-most projections 104a, 104b preferably are disposed at an angle δ of approximately 67 degrees from the center plane B-B, and thus about 134 degrees from each other. Accordingly, projections 104a, 104b are each disposed at an angle λ of approximately 113 degrees from center projection 104c, which is located on the plane B-B. This configuration provides a secure and stable clamping connection between the clamp 100 and the object 200. The projections could be spaced-apart by different amounts, for example, they could be equally-spaced about the hoop section 102 by approximately 120 degrees. Having the end-most projections 104a, 104b spaced-apart from each other on the hoop section 102 by more than 120 degrees, as shown in the illustrated example, creates a larger gap between the projections 104a, 104b than would be created if the projections 104a, 104b were spaced from each other by 120 degrees or less. This may be desirable as it will limit the amount of radial expansion of the hoop section 102 than would otherwise be necessary in order to pass the object 200 to be clamped through the gap between the projections 104a, 104b. Limiting the amount of radial expansion necessary minimizes the stresses created during clamp installation and limits the amount of actuating force required to expand the hoop sufficiently for installation. This, of course, has to be balanced with the objective of keeping the barrel retained within the clamp, which makes it desirable to locate projections 104a and 104b at an angle greater than 90 degrees from projection104c.

Extensions 110a, 110b provide a hard-stop feature to prevent over-flexing of the clamp 100. Specifically, extensions 110a, 110b limit the maximum deformation and stress in the hoop section 102 by limiting the range of motion of the exterior arms 108a and 108b. As explained above, when installing the clamp 100 onto or removing the clamp 100 from a barrel 200, force is applied to the exterior arms 108a and 108b to force the two members toward each other, thereby opening the hoop section 102 and moving projections 104a and 104b away from each other a minimum distance to allow space for the clamp 100 to be placed on or removed from the object 200.

It is preferred that the hoop section 102 is not stressed beyond its elastic limit. If the hoop section 102 were stressed beyond its elastic limit, thereby causing the hoop material to yield, the clamping force that could be generated by the hoop section 102 on the object being clamped would be affected. Extensions 110a, 110b extend toward one another from each respective exterior arm 108a, 108b. In the illustrated example, the extensions 110a, 110b extend from the ends of the arms 108a, 108b opposite the elbows 106a, 106b. The distance between opposed end faces 111a and 111b of the extensions 110a and 110b, respectively, is set based on the geometry, dimensions, and material of the clamp so as to prevent yielding in the hoop section 102 while allowing sufficient expansion of the hoop section 102 so as to permit clamp installation on an object. Over-expansion of the hoop section 102 of the clamp 100 is prevented when installing the clamp 100, because the extensions 110a, 110b will strike each other to provide a hard-stop and prevent any further movement of the exterior arms 108a, 108b toward each other, thereby preventing any further expansion of the hoop section 102. Thus, the extensions 110a and 110b define limitation means which ensure that the hoop section 102 will not be over-expanded during installation.

In the illustrated embodiment, extensions 110a and 110b are substantially perpendicular to the plane B-B and are generally co-aligned when no external forces are being applied the clamp 100. The opposed faces 111a, 111b are formed at slight angles relative to a longitudinal dimension of the extensions. As the exterior arms 108a, 108b are urged toward one another, the extensions 110a, 110b will no longer be co-aligned and perpendicular to the plane of symmetry B-B. The faces 111a, 111b are angled so that when the extensions 110a, 110b contact each other at faces 111a, 111b, the faces 111a, 111b will be substantially parallel to each other, thereby providing surface contact, as opposed to point contact, between the faces 111a, 111b.

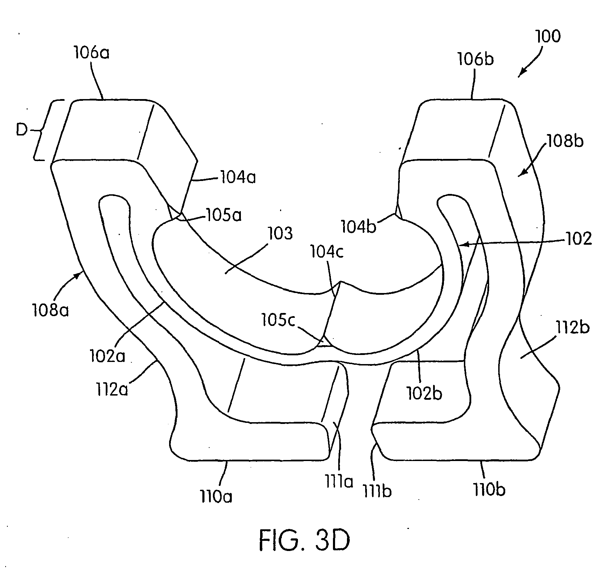

Figs. 3C and 3D show a cross-sectional view and perspective view, respectively, of clamp 100. As shown, the clamp has a depth D that can be small in comparison to the radius R of the hoop section 102.

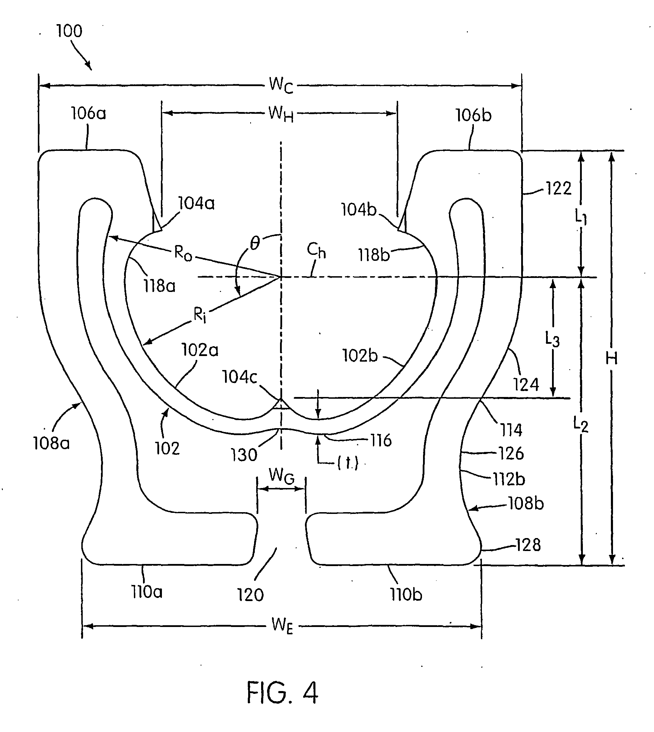

The thickness (t) (see Fig. 4) of the hoop section 102 is selected, depending on the material of which the hoop section 102 is manufactured, to produce a desired gripping force and a desired actuation force (i.e., amount to force required to open the clamp). The thickness (t) of the hoop section is preferably variable and is generally less than that of the exterior arms 108a, 108b, so that when the exterior arms 108a, 108b are squeezed toward each other, the hoop section 102, and not the exterior arms 108a, 108b, flexes. The thickness of the hoop section 102 is described in more detail below.

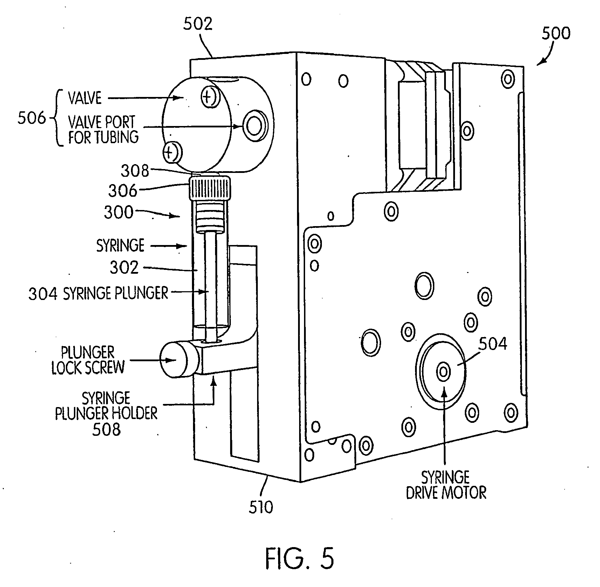

Fig. 5 show an exemplary syringe pump 500 of the type on which the clamp 100 might be employed. Syringe pump 500 includes a housing 502, a motor 504 contained within the housing 502, a valve assembly 506, a syringe 300 operatively coupled to the valve assembly 506, and a movable arm 508 operatively coupled to both the motor 504 and the syringe 300. The syringe 300 includes a glass barrel 302, a plunger 304 disposed within the barrel 302, and a hub 306 with a projecting nipple 308 (partially shown) which is threaded for connecting the barrel 200 with the valve assembly 506. Pumping is effected by movement of the plunger 304 by the movable arm 508 powered by the motor 504. Exemplary syringe pumps on which the clamp can be installed as an anti-rotation device include the model XP3000 Modular Digital Pump available from Cavro Scientific Instruments, Inc. of San Jose, California, and the PSD/4 syringe pump, model 7858-04 available from Hamilton Company of Reno, Nevada.

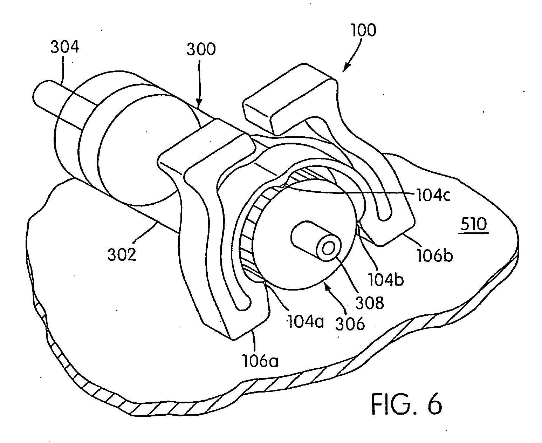

As was explained briefly in the Background section above, repeated cycling of the plunger 304 during operation of the syringe pump 500 and/or vibration of an instrument on which the syringe pump 500 is installed can cause the barrel 302 and/or the hub306 to rotate, thereby causing the nipple 308 to be loosened with respect to the valve assembly 506. As shown in partial view in Fig. 6, the clamp 100 is secured to the hub 306 of the syringe 300, and elbows 106a, 106b of the clamp 100 contact the end surface 510 of the housing 502 - while the anti-slipping elements (e.g., protrusions 104a, 104b, 104c) prevent rotation of the clamp 100 with respect to the hub 306 - to prevent rotation of the barrel 302 and the hub 306. In other arrangements, one or both elbows 106a, 106b, some other portion of the clamp 100, or one or more extensions attached to the clamp 100 contact some portion of the pump (other than its housing) and/or some other adjacent structure of an instrument on which the pump is installed to prevent rotation of the clamp and thereby prevent rotation of the syringe on which the clamp is installed.

Although the clamp 100 is installed on the hub 306, which is not made of glass, excessive clamping forces applied to the hub can be transmitted to the glass barrel 302, thereby causing the barrel 302 to crack. When the clamp 100 is employed on a syringe pump, such as syringe pump 500, the depth D (See Fig. 3C) may be equal to or less than the axial length of the hub 306, in order to prevent the clamp from riding on the glass barrel 302. The depth D of the clamp 100 may also be greater than the axial length of the hub 306, especially when the diameter of the barrel 302 in the region adjacent the hub 306 is less than the diameter of the hub 306.

It can be appreciated from Figs. 5-6 that the arrangement of the clamp 100 allows it to be installed on a tubular structure, such as syringe 300, which is closely adjacent to a structure (such as housing 502 of the syringe pump 500) which might otherwise interfere with use of a conventional clamp, such as either of the clamps shown in Figs. 1 and 2.

Also, the clamp 100 can be installed laterally onto the syringe 800 by expanding the hoop section 102 and placing the clamp onto the syringe 300 while the syringe is installed on the syringe pump 500. Prior art clamps, such as those shown in Figs. 1 and 2, do not include an open hoop section and thus must be installed by slipping the clamp over one of the ends of the syringe 300. Thus, to install the prior art clamp on the syringe 300, it is necessary to disconnect the syringe 300 from either the valve assembly 506 or the movable arm 508 to permit the clamp to be slipped onto the hub 306.

Clamp 100 is preferably fabricated from a single piece of material, such as plastic or metal, but may be a composite, such as a metal core over-molded with an elastomer. The material should inherently generate the elastic forces necessary to provide adequate clamping force (gripping force) without loosening over time, but without creating an excessive clamping force which can damage an object being clamped. In an exemplary embodiment of the syringe pump assembly of the present invention, the clamp is designed to exert gripping forces of 2-5 pounds, while requiring a force of 3 - 7 pounds to actuate (i.e., open) the clamp.

Plastics are not suitable for some applications because of their dimensional instability due to material creep under prolonged stress, although it is contemplated that a plastic with satisfactory characteristics could be used for some applications, particularly where clamping of a long duration is not required. Metals, such as aluminum, are the preferred material. Aluminum 7075-T6 is most preferred, because it has a much higher yield stress than more standard aluminum alloys, such as aluminum 6061-T6, as shown in the table below:

| Material | Yield Stress (psi) | Modulus of Elasticity (ksi) |
|---|---|---|
| Noryl | 14,400 | 363 |
| Aluminum 6061-T6 | 39,300 | 10,000 |
| Aluminum 7075-T6 | 73,200 | 10,400 |

Fig. 4 shows clamp 100 having exemplary dimensions of a preferred shape for a clamp manufactured from aluminum and using milling techniques known to those skilled in the art. The specific dimensions described below are for a clamp 100 designed to hold a 5 ml syringe (not shown in Fig 4) with a hub having a diameter of about 0.720 inches. The dimensions are described to provide exemplary values of the dimensions themselves and exemplary values of size ratios for various portions of the clamp.

For such a clamp, the overall width $W_c$ of the clamp is 1.34 inches across the top (elbows 106a, 106b), and $W_e$ is 1.144 inches across the bottom (extensions 110a, 110b). The overall height H of the clamp is 1.225 inches, and the overall depth D (See Fig. 3C) is 0.25 inches. Exterior arm 108b has an initial straight section 122 extending a length $L_1$, approximately 0.375 inches, from the top of elbow 106b to a point corresponding to the location of a horizontal axis ($C_h$) passing through the center of the cavity defined by hoop section 102 (i.e., the center of object 200 in Fig. 3A). The initial straight section 122 is followed by a first curved section 124 that substantially follows the shape of corresponding arcuate section 102b. The first curved section 124 is followed - beginning approximately at point 114 - by a second curved section 126 having a curvature reversed from the first curved section 124. The second curved section 126, which terminates at a heal portion 128, defines the finger grip 112b. A distance $L_2$ from the bottom surface of the clamp (extension 110b) to axis $C_h$ is approximately 0.85 inches. A distance $L_3$ from the top of projection 104c to axis $C_h$ is approximately 0.34 inches. Arm 108a is a mirror image replica of arm 108b.

A gap 120 between the extensions 110a and 110b has a length $W_G$, approximately 0.132 inches. The thickness (t) of the hoop section 102 varies from a point of minimum thickness at point 116 (at which the thickness (t) is about 0.039 inches in this example) and becomes gradually thicker for each arcuate section 102a, 102b from point 116 to the ends 118a, 118b of hoop section 102.

The thickness (t) of the hoop section 102 preferably varies in a manner such that the bending stresses in the hoop section 102 are uniformly distributed and such that peak stresses in the hoop section 102 are minimized. In an exemplary embodiment, the thickness of the hoop section is determined generally by the formula:

$$t = R_o - R_i - (b * \sqrt{\sin\Theta})$$

where: t is the local thickness of the hoop section 102; $R_o$ is the radial distance to the exterior of the hoop section 102 at its thickest section; $R_i$ is the constant inner radius of the hoop section 102; b is the difference between the maximum and minimum thicknesses along the hoop section 102; and $\Theta$ is the angular position along the hoop section 102 measured from horizontal axis $C_h$, as illustrated in Fig. 4.

During installation of the clamp 100, when the exterior arms 108a, 108b are moved toward each other, stress in the hoop section 102 is due to the flexure caused by the exterior arms. When the clamp 100 is installed, forces are not exerted on the exterior arms 108a, 108b, but at the contact points (e.g., radial projections 104a-c) of the clamp 100. These conditions result in peak stresses at the bottom of the hoop section 102, in the region of projection 104c. The material condition in this region can be adjusted to reduce these stresses. In the preferred embodiment, a depression 130 is formed in the hoop section 102 opposite the projection 104c. As a result of the design, it has been determined that the peak stress experienced by the clamp 100 in the installed state will be lower than that experienced by the clamp during installation. Note that the above thickness formula only applies in the regions of the hoop section 102 on each side of the center protrusion 104c between the center protrusion 104c and each of the opposing protrusions 104a, 104b, excluding the transition region around the depression 130 and any blending radii between sections.

Elastic analysis of structures, such as Finite Element Method (FEM), may be employed to estimate resultant stresses and displacements from applied loads. Successive iterations of a computer aided design (CAD) model and FEM calculations may be performed to achieve a structural design which achieves the features of the present invention.

To estimate the stresses that the clamp will experience, constraints and forces were applied in the FEM model in two ways: a first load case considers the clamp at maximum deflection during actuation, and a second load case considers the clamp once it is in place on an object of a given radius. Of course, depending on the object to be clamped, the desired actuating and gripping forces, and the material to be used, the process of CAD modeling and FEM calculations may produce a clamp having a structure that varies from the embodiments shown and described herein.

The displacement, or deformation, of the clamp can also be computed to assure that the clamp will open wide enough around a syringe, barrel, or other object to be clamped during installation and to assure that the extensions 110a, 110b will make contact with each other before the hoop section 102 can be yielded. The design can be further iterated to achieve these displacement values while keeping the amount of required force within an acceptable range. The iterative process of adjusting the CAD modeling based on subsequent FEM calculations results in the rapid convergence to a design that provides the advantages and characteristics of the present invention.

In analyzing and designing the clamp suitable for the present invention, the inventors employed Cosmos Designstar 3.0 (Structural Research & Analysis Corp., Los Angeles, CA) and Mechanica with Pro/Engineer 2001 (Parametric Technologies Corp., Waltham, MA) for all FEM analyses. Grid refinement studies were performed to ensure grid independent solutions.

Stress analysis of structures is described in further detail in: Foundations of Solid Mechanics, Y.C. Fung, 1965, Prentice-Hall, ISN 0-13-329912-0; Advanced Strength and Applied Elasticity, A.C. Ugural and S.K. Fenster, 1975, Elsevier, ISBN 0-444-00160-3; and Formulas for Stress and Strain (5th Edition), R.J. Roark and W.C. Young, 1975, McGraw-Hill, ISBN 0-07-053031-9.

While the invention has been described in connection with what are presently considered to be the most practical and preferred embodiments, it is to be understood that the invention is not to be limited to the disclosed embodiments, but, on the contrary, is intended to cover various modifications and equivalent arrangements included within the scope of the appended claims.

**Claims**

1. A syringe pump assembly comprising:

   a mechanized syringe (300) including a barrel (302), a plunger (304) disposed within said barrel (302) for reciprocating movement therein, and a motor (504) operatively coupled to said plunger (304) for effecting mechanized movement of said plunger (304); **characterized by**
   an anti-rotation clamp (100) secured to said syringe (300) and constructed and arranged to prevent rotation of said syringe by contacting a structure adjacent to said syringe, said anti-rotation clamp comprising:
   a hoop section (102) forming a portion of a generally circular closed loop with first and second opposed ends (118a, 118b) defining a gap therebetween, said hoop section being constructed and arranged to generate a clamping force when placed on said syringe to secure said clamp to said syringe; and
   first and second actuating elements (108a, 108b), each having first ends and second ends (111a, 111b), said first ends of said first and second actuating elements (108a, 108b) being coupled to said first and second ends (118a, 118b), respectively, of said hoop section (102), said first and second actuating elements (108a, 108b) being spaced apart from and extending along opposite sides of said hoop section (102), said second ends (111a, 111b) of said actuating elements (108a, 108b) being in opposed, spaced-apart relation with respect to each other, and said actuating elements (108a, 108b) being constructed and arranged to cause said hoop section (102) to expand when said second ends (111a, 111b) of said first and second actuating elements are moved toward each other, thereby increasing the size of said gap.

2. The syringe pump assembly of claim 1, further comprising anti-slipping elements disposed on an Interior surface (103) of said hoop section (102) and constructed and arranged to resist slippage of said hoop section (102) with respect to a clamped object.

3. The syringe pump assembly of claim 2, wherein said anti-sllpping elements comprise a plurality of circumferentially spaced radial projections formed on said interior surface (103) of said hoop section (102).

4. The syringe pump assembly of claim 3, wherein said plurality of projections consists of first, second, and third projections (104a, 104b, 104c),

5. The syringe pump assembly of claim 4, wherein said first and second projections (104a, 104b) are disposed at said first and second ends (118a, 118b), respectively, of said hoop section (102) and said third projection (104c) is disposed at a mid-point between said first and second projections (104a, 104b).

6. The syringe pump assembly of one of claims 1 to 5, wherein said clamp (100) is substantially symmetrical about a center plane bisecting the gap between said first and second ends (118a, 118b) of said hoop section (102) and bisecting a gap between said second ends (111a, 111b) of said actuating elements (108a, 108b).

7. The syringe pump assembly of claim 6, wherein said clamp further comprises first and second extensions (110a, 110b) extending toward each other from said first and second actuating elements (108a, 108b), respectively, and positioned In an opposed, spaced-apart relation with respect to each other, such that when said actuating elements (108a, 108b) are moved toward one another, said first and second extensions (110a, 110b) come in contact with each other to prevent yielding of said hoop section (102), wherein each extension (110a, 110b) comprises a first end attached to an end of the respective actuating element (108a, 108b) and a second end (111a, 111b), and wherein said first and second extensions (110a, 110b) are generally perpendicular to the center plane when said first and second actuating elements are not actuated and extend laterally toward each other in general alignment with each other and with the respective second ends (111a, 111b) of the first and second extensions (110a, 110b) positioned in opposed, spaced-apart relation with respect to each other.

8. The syringe pump assembly of any of claims 1 to 7, wherein a thickness of said hoop section (102) varies circumferentially.

9. The syringe pump assembly of claim 8, wherein the thickness of said hoop section (102) decreases from each of said first and second opposed ends (118a, 118b) of said hoop section (102).

10. The syringe pump assembly of any of claims 1 to 9, wherein said clamp (100) is fabricated from a single piece of material.

11. The syringe pump assembly of claim 10, wherein said material comprises a metal.

12. The syringe pump assembly of claim 11, wherein the metal comprises an aluminum alloy.

13. The syringe pump assembly of any of claims 1 to 12, further comprising first and second elbows (106a, 106b) coupling said first and second actuating elements (108a, 108b) to said first and second ends (118a, 118b), respectively, of said hoop section (102).

14. The syringe pump assembly of any of claims 1 to 13, wherein the shape of a portion of each of said first and second actuating elements (108a, 108b) extending from said first ends thereof generally corresponds to the shape of said hoop section (102).

15. The syringe pump assembly of any of claims 1 to 14, wherein each of said first and second actuating elements (108a, 108b) includes a curved, finger-grip portion.

16. The syringe pump assembly of any of claims 1 to 15, said syringe (300) further including a hub (306) connected to one end of said barrel (302), wherein said clamp (100) is secured to said hub (306).

17. The syringe pump assembly of any of claims 1 to 16, wherein a clamping force generated by said hoop section (102) on said syringe (300) is limited by the restoring forces inherent in said hoop section (102) when said hoop section (102) is placed on a syringe (300) having an outside dimension that is larger than an inside dimension of said hoop section (102).

**Patentansprüche**

1. Spritzenpumpen-Anordnung, aufweisend:

eine mechanisierte Spritze (300), die einen Zylinder (302), einen Kolben (304), der in dem Zylinder (302) für eine Auf- und Abwärts-Bewegung in diesem angeordnet ist, und einen Motor (504) aufweist, der zum Ausführen einer mechanisierten Bewegung des Kolbens (304) operativ mit dem Kolben (304) gekuppelt ist; **gekennzeichnet durch**
eine Anti-Rotations-Klemme (100), die an der Spritze (300) befestigt und so konstruiert und angeordnet ist, dass sie eine Rotation der Spritze **durch** Kontaktieren einer Struktur benachbart zu der Spritze verhindert, wobei die Anti-Rotations-Klemme (100) aufweist:
einen Bügelabschnitt (102), der einen Abschnitt einer im Wesentlichen kreisförmigen geschlossenen Schlaufe bildet und ein erstes und ein zweites Ende (118a, 118b) aufweist, die einander gegenüberliegen und dazwischen einen Spalt definieren, wobei der Bügelabschnitt so konstruiert und angeordnet ist, dass er eine Klemmkraft erzeugt, wenn er auf der Spritze platziert wird, so dass die Klemme an der Spritze befestigt wird, und
ein erstes und ein zweites Betätigungselement (108a, 108b), das jeweils ein erstes und ein zweites Ende (111a, 111b) aufweist, wobei das jeweilige erste Ende des ersten und des zweiten Betätigungselements (108a, 108b) mit dem ersten beziehungsweise dem zweiten Ende (118a, 118b) des Bügelabschnitts (102) verbunden ist, wobei sich das erste und das zweite Betätigungselement (108a, 108b) im Abstand von dem Bügelabschnitt (102) entlang dessen entgegengesetzten Seiten erstrecken, wobei die zweiten Enden (111a, 111b) der Betätigungselemente (108a, 108b) einander gegenüberliegend im Abstand voneinander angeordnet sind, und wobei die Betätigungselemente (108a, 108b) so konstruiert und angeordnet sind, dass sie bewirken, dass sich der Bügelabschnitt (102) ausdehnt, wenn die zweiten Enden (111a, 111b) des ersten und des zweiten Betätigungselements in Richtung zueinander bewegt werden, wodurch die Größe des Spalts erhöht wird.

2. Spritzenpumpen-Anordnung gemäß Anspruch 1, die ferner Anti-Rutsch-Elemente aufweist, die an einer Innenfläche (103) des Bügelabschnitts (102) positioniert und so konstruiert und angeordnet sind, dass sie einem Rutschen des Bügelabschnitts (102) relativ zu einem festgeklemmten Objekt widerstehen.

3. Spritzenpumpen-Anordnung gemäß Anspruch 2, wobei die Anti-Rutsch-Elemente eine Mehrzahl von umlaufend im Abstand angeordneten radialen Vorsprüngen aufweisen, die auf der Innenfläche (103) des Bügelabschnitts (102) ausgebildet sind.

**4.** Spritzenpumpen-Anordnung gemäß Anspruch 3, wobei die Mehrzahl von Vorsprüngen aus einem ersten, einem zweiten und einem dritten Vorsprung (104a, 104b, 104c) besteht.

**5.** Spritzenpumpen-Anordnung gemäß Anspruch 4, wobei der erste und zweite Vorsprung (104a, 104b) an dem ersten beziehungsweise zweiten Ende (118a, 118b) des Bügelabschnitts (102) angeordnet sind, und der dritte Vorsprung (104c) an einem Mittelpunkt zwischen dem ersten und zweiten Vorsprung (104a, 104b) angeordnet ist.

**6.** Spritzenpumpen-Anordnung gemäß einem der Ansprüche 1 bis 5, wobei die Klemme (100) um eine Mittelebene, die den Spalt zwischen dem ersten und dem zweiten Ende (118a, 118b) des Bügelabschnitts (102) in zwei Hälften teilt, und die einen Spalt zwischen den zweiten Enden (111a, 111b) der Betätigungselemente (108a, 108b) in zwei Hälften teilt, im Wesentlichen symmetrisch ist.

**7.** Spritzenpumpen-Anordnung gemäß Anspruch 6, wobei die Klemme ferner einen ersten und einen zweiten Verlängerungsabschnitt (110a, 110b) aufweist, die sich in Richtung zueinander von dem ersten beziehungsweise zweiten Betätigungselement (108a, 108b) erstrecken, und die voneinander im Abstand und einander gegenüberliegend derart angeordnet sind, dass der erste und der zweite Verlängerungsabschnitt (110a, 110b) in Kontakt miteinander kommen, wenn die Betätigungselemente (108a, 108b) in Richtung zueinander bewegt werden, so dass ein Nachgeben des Bügelabschnitts (102) verhindert wird, wobei jeder Verlängerungsabschnitt (110a, 110b) ein erstes Ende, das an einem Ende des jeweiligen Betätigungselements (108a, 108b) angebracht ist, und ein zweites Ende (111a, 111b) aufweist, und wobei der erste und der zweite Verlängerungsabschnitt (110a, 110b) im Wesentlichen senkrecht zu der Mittelebene sind, wenn das erste und das zweite Betätigungselement nicht betätigt werden, und sich lateral in Richtung zueinander und insgesamt aufeinander ausgerichtet erstrecken, während die jeweiligen zweiten Enden (111a, 111b) des ersten und des zweiten Verlängerungsabschnitts (110a, 11b) einander gegenüberliegend und im Abstand zueinander positioniert sind.

**8.** Spritzenpumpen-Anordnung gemäß einem der Ansprüche 1 bis 7, wobei eine Dicke des Bügelabschnitts (102) in Umlaufrichtung variiert.

**9.** Spritzenpumpen-Anordnung gemäß Anspruch 8, wobei sich die Dicke des Bügelabschnitts (102) ausgehend von jedem von dem ersten und dem zweiten gegenüberliegend angeordneten Ende (118a, 118b) des Bügelabschnitts (102) verringert.

**10.** Spritzenpumpen-Anordnung gemäß einem der Ansprüche 1 bis 9, wobei die Klemme (100) aus einem einzigen Materialstück hergestellt ist.

**11.** Spritzenpumpen-Anordnung gemäß Anspruch 10, wobei das Material ein Metall aufweist.

**12.** Spritzenpumpen-Anordnung gemäß Anspruch 11, wobei das Metall eine Aluminium-Legierung aufweist.

**13.** Spritzenpumpen-Anordnung gemäß einem der Ansprüche 1 bis 12, ferner ein erstes und ein zweites Bogenstück (106a, 106b) aufweisend, die das erste und das zweite Betätigungselement (108a, 108b) mit dem ersten beziehungsweise dem zweiten Ende (118a, 118b) des Bügelabschnitts (102) verbinden.

**14.** Spritzenpumpen-Anordnung gemäß einem der Ansprüche 1 bis 13, wobei die Form eines Abschnitts von jedem von dem ersten und dem zweiten Betätigungs-Element (108a, 108b), der sich von den ersten Enden davon erstreckt, insgesamt der Form des Bügelabschnitts (102) entspricht.

**15.** Spritzenpumpen-Anordnung gemäß einem der Ansprüche 1 bis 14, wobei jedes von dem ersten und dem zweiten Betätigungselement (108a, 108b) einen gekrümmten Finger-Greifabschnitt aufweist.

**16.** Spritzenpumpen-Anordnung gemäß einem der Ansprüche 1 bis 15, wobei die Spritze (300) eine Nabe (306) aufweist, die mit einen Ende des Zylinders (302) verbunden ist, wobei die Klemme (100) an der Nabe (306) befestigt ist.

**17.** Spritzenpumpen-Anordnung gemäß einem der Ansprüche 1 bis 16, wobei eine Klemmkraft, die von dem Bügelabschnitt (102) auf die Spritze (300) ausgeübt wird, durch die in dem Bügelabschnitt (102) inhärenten Rückstellkräfte begrenzt ist, wenn der Bügelabschnitt (102) auf einer Spritze (300) platziert wird, deren Außenabmessung größer ist als eine Innenabmessung des Bügelabschnitts (102).

**Revendications**

1. Ensemble de pompe de seringue comprenant :

une seringue mécanisée (300) comprenant un cylindre (302), un piston (304) disposés dans ledit cylindre (302) pour un mouvement de va-et-vient dans celui-ci, et un moteur (504) fonctionnellement couplé audit piston (304) pour effectuer le mouvement mécanisé dudit piston (304) ; **caractérisé par**
un étrier anti-rotation (100) fixé à ladite seringue (300) et construit et agencé pour empêcher la rotation de ladite seringue en mettant en contact une structure adjacente à ladite seringue, ledit étrier anti-rotation comprenant :
une section circulaire (102) formant une partie d'une boucle fermée généralement circulaire avec des premières et deuxièmes extrémités opposées (118a, 118b) définissant un espace entre celles-ci, ladite section circulaire étant construite et agencée pour générer une force de serrage lorsqu'elle est placée sur ladite seringue pour fixer ledit étrier à ladite seringue ; et
des premier et deuxième éléments d'actionnement (108a, 108b), ayant chacun des premières extrémités et des deuxièmes extrémités (111a, 111b), lesdites premières extrémités desdits premier et deuxième éléments d'actionnement (108a, 108b) étant couplées auxdites premières et deuxièmes extrémités (118a, 118b), respectivement, de ladite section circulaire (102), lesdits premier et deuxième éléments d'actionnement (108a, 108b) étant espacés et s'étendant le long des côtés opposés de ladite section circulaire (102), lesdites deuxièmes extrémités (111a, 111b) desdits éléments d'actionnement (108a, 108b) étant opposé es, espacées l'une par rapport à l'autre, et lesdits éléments d'actionnement (108a, 108b) étant construits et agencés pour amener ladite section circulaire (102) à s'élargir lorsque lesdites deuxièmes extrémités (111a, 111b) desdits premier et deuxième éléments d'actionnement sont déplacées l'une vers l'autre, augmentant ainsi la taille dudit espace.

2. Ensemble de pompe de seringue selon la revendication 1, comprenant en outre des éléments d'anti-glissement disposés sur une surface intérieure (103) de ladite section circulaire (102) et construits et agencés pour résister au glissement de ladite section circulaire (102) par rapport à un objet serré.

3. Ensemble de pompe de seringue selon la revendication 2, dans lequel lesdits éléments d'anti-glissement comprennent une pluralité de saillies radiales espacées circonférentiellement formées sur ladite surface intérieure (103) de ladite section circulaire (102).

4. Ensemble de pompe de seringue selon la revendication 3, dans lequel ladite pluralité de saillies comprend des première, deuxième, et troisième saillies (104a, 104b, 104c).

5. Ensemble de pompe de seringue selon la revendication 4, dans lequel lesdites première et deuxième saillies (104a, 104b) sont disposées auxdites premières et deuxièmes extrémités (118a, 118b), respectivement, de ladite section circulaire (102) et ladite troisième saillie (104c) est disposée à un point médian entre lesdites première et deuxième saillies (104a, 104b).

6. Ensemble de pompe de seringue selon l'une des revendications 1 5, dans lequel ledit étrier (100) est sensiblement symétrique par rapport à un plan central bissectant l'espace entre lesdites premières et deuxièmes extrémités (118a, 118b) de ladite section circulaire (102) et bissectant un espace entre lesdites deuxièmes extrémités (111a, 111b) desdits éléments d'actionnement (108a, 108b).

7. Ensemble de pompe de seringue selon la revendication 6, dans lequel ledit étrier comprend en outre des première et deuxième extensions (110a, 110b) s'étendant l'une vers l'autre à partir desdits premier et deuxième éléments d'actionnement (108a, 108b), respectivement, et positionnées de manière opposée et espacée l'une par rapport à l'autre, de sorte que lorsque lesdits éléments d'actionnement (108a, 108b) se déplacent l'un vers l'autre, lesdites première et deuxième extensions (110a, 110b) entrent en contact l'une avec l'autre pour empêcher l'infléchissement de ladite section circulaire (102), où chaque extension (110a, 110b) comprend une première extrémité attachée à une extrémité de l'élément d'actionnement respectif (108a, 108b) et une deuxième extrémité (111a, 111b), et où lesdites première et deuxième extensions (110a, 110b) sont généralement perpendiculaires au plan central lorsque lesdits premier et deuxième éléments d'actionnement ne sont pas actionnés et s'étendent latéralement l'un vers l'autre sensiblement en alignement l'un avec l'autre et avec les deuxièmes extrémités respectives (111a, 111b) des première et deuxième extensions (110a, 110b) positionnées de manière opposée et espacée l'une par rapport à l'autre.

8. Ensemble de pompe de seringue selon l'une quelconque des revendications 1 à 7, dans lequel une épaisseur de ladite section circulaire (102) varie circonférentiellement.

9. Ensemble de pompe de seringue selon la revendication 8, dans lequel l'épaisseur de la section circulaire (102) décroît à partir de chacune desdites première et deuxième extrémités opposées (118a, 118b) de ladite section circulaire (102).

10. Ensemble de pompe de seringue selon l'une quelconque des revendications 1 à 9, dans lequel ledit étrier (100) est fabriqué d'une seule pièce de matériau.

11. Ensemble de pompe de seringue selon la revendication 10, dans lequel ledit matériau est composé d'un métal.

12. Ensemble de pompe de seringue selon la revendication 11, dans lequel ledit métal consiste en un alliage d'aluminium.

13. Ensemble de pompe de seringue selon l'une quelconque des revendications 1 à 12, comprenant en outre des premier et deuxième coudes (106a, 106b) couplant lesdits premier et deuxième éléments d'actionnement (108a, 108b) auxdites premières et deuxièmes extrémités (118a, 118b), respectivement, de ladite section circulaire (102).

14. Ensemble de pompe de seringue selon l'une quelconque des revendications 1 à 13, dans lequel la forme d'une partie de chacun desdits premier et deuxième éléments d'actionnement (108a, 108b) s'étendant à partir desdites premières extrémités de ces derniers correspond de manière générale à la forme de ladite section circulaire (102).

15. Ensemble de pompe de seringue selon l'une quelconque des revendications 1 à 14, dans lequel chacun desdits premier et deuxième éléments d'actionnement (108a, 108b) comprend une partie de préhension courbée.

16. Ensemble de pompe de seringue selon l'une quelconque des revendications 1 à 15, ladite seringue (300) comprenant en outre un moyeu (306) relié à l'une des extrémités dudit cylindre (302), où ledit étrier (100) est fixé audit moyeu (306).

17. Ensemble de pompe de seringue selon l'une quelconque des revendications 1 à 16, dans lequel une force de serrage générée par ladite section circulaire (102) sur ladite seringue (300) est limitée par les forces de rappel inhérentes à ladite section circulaire (102) lorsque ladite section circulaire (102) est placée sur une seringue (300) ayant une dimension extérieure qui est supérieure à une dimension intérieure de ladite section circulaire (102).

FIG.1
PRIOR ART

32

30

42    40

38

44

36  46  34

FIG. 2
PRIOR ART

FIG. 3A

106a

104a

105a

102a

$\delta=67°$

$\lambda$

B

FIG. 3B

100

106a

105a

104a

102a

R

104c

105c

110a

D

FIG. 3C

FIG. 3D

FIG. 4

502

500

VALVE
506 { VALVE PORT
FOR TUBING

308
306
300

SYRINGE

302

304 SYRINGE PLUNGER

PLUNGER
LOCK SCREW

SYRINGE
PLUNGER HOLDER
508

504

510

SYRINGE
DRIVE MOTOR

FIG. 5

FIG. 6

**EP 1 660 156 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CH 269955
- FR 2278006 A
- US 2002173809 A
- US 5219099 A

### Non-patent literature cited in the description

- **Y.C. FUNG.** Foundations of Solid Mechanics. Prentice-Hall, 1965
- **A.C. UGURAL ; S.K. FENSTER.** Advanced Strength and Applied Elasticity. Elsevier, 1975
- **R.J. ROARK ; W.C. YOUNG.** Stress and Strain. McGraw-Hill, 1975